(19) **Europäisches Patentamt / European Patent Office / Office européen des brevets**

(11) **EP 4 696 701 A1**

(12) **EUROPEAN PATENT APPLICATION**
published in accordance with Art. 153(4) EPC

(43) Date of publication:
**18.02.2026 Bulletin 2026/08**

(21) Application number: **24788028.9**

(22) Date of filing: **08.04.2024**

(51) International Patent Classification (IPC):
**C07K 14/00** (2006.01)   **C12N 5/00** (2006.01)
**C12N 1/20** (2026.01)

(52) Cooperative Patent Classification (CPC):
**C07K 14/00; C12N 1/16; C12N 1/20; C12N 5/00; C12N 5/06; C12N 15/11;** C12R 2001/19; C12R 2001/865

(86) International application number:
**PCT/CN2024/086601**

(87) International publication number:
**WO 2024/212925 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.04.2023   CN 202310391903**

(71) Applicant: **Zhang, Jing**
**Gusu District**
**Suzhou, Jiangsu 215000 (CN)**

(72) Inventors:
• **JIN, Wenbo**
  **Suzhou, Jiangsu 215000 (CN)**
• **LIU, Jianning**
  **Suzhou, Jiangsu 215000 (CN)**
• **ZHANG, Jing**
  **Suzhou, Jiangsu 215000 (CN)**

(74) Representative: **Schiweck Weinzierl Koch Patentanwälte Partnerschaft mbB**
**Ganghoferstraße 68 B**
**80339 München (DE)**

Remarks:
The complete document including Reference Table(s) and the Sequence Listing(s) can be downloaded from the EPO website

(54) **PROTEIN, AND CULTURE MEDIUM CONTAINING SAME AND USE THEREOF**

(57)   Provided are a protein, and a culture medium containing same and the use thereof. The protein is a variant of an amino acid sequence as shown in SEQ ID NO: 1. When the protein is added to the culture medium, the culture medium can effectively stimulate the potential of cells or microorganisms, improve the growth rate thereof and maintain the vitality thereof under sudden adverse conditions.

EP 4 696 701 A1

**Description**

**FIELD OF THE INVENTION**

The present invention belongs to the field of biotechnology, in particular relates to a protein, and further relates to a culture medium containing the protein and the use thereof in culturing cells or microorganisms.

**BACKGROUND OF THE INVENTION**

In cell culture and microbial fermentation, culture medium, as a nutrient for cells, is essential for cell growth. At present, there are various methods to optimize the culture medium to improve the efficiency of cell culture. However, there is still a need for a culture medium that can stimulate the potential of cells or microorganisms, improve the growth rate of cells or microorganisms, and maintain the vitality of cells or microorganisms under sudden adverse conditions.

**SUMMARY OF THE INVENTION**

Therefore, it is an objective of the present invention to provide a protein for cell or microorganism culture on the basis of the prior art. The protein of the present invention can be used as an additive of the cell culture medium, and can significantly promote the survival and growth of cells or microorganisms.

The objective of the present invention is achieved through the following technical solutions:

The first aspect of the present invention provides a protein, which is a variant of an amino acid sequence as shown in SEQ ID NO: 1, and the variant at least comprises one of the following mutations:

(a) S is added to the N-terminus;
(b) position 4 is H, G, Q, K or E;
(c) position 5 is D, H, G, S, A, T or is absent;
(d) position 6 is V, I, P, N, A or E;
(e) position 7 is Q, H, P or is absent;
(f) position 8 is N, H or D;
(g) position 9 is M;
(h) position 10 is D;
(i) position 11 is S or G;
(j) position 12 is S or E;
(k) position 13 is K;
(l) position 14 is M;
(m) position 15 is G or D;
(n) position 16 is S, T or A;
(o) position 18 is G;
(p) position 23 is C;
(q) position 25 is T;
(r) position 28 is R;
(s) position 32 is D;
(t) position 38 is Q, K, R, N or S;
(u) position 39 is Y;
(v) position 46 is N or T;
(w) position 49 is V;
(x) position 53 is K;
(y) position 54 is N;
(z) position 56 is K;

(aa) position 59 is K or R;
(bb) position 63 is F; and/or
(cc) the C-terminus is pentapeptide GLVPR or is absent.

The present invention is based on the inventor's unexpected observation that these proteins can stimulate the survival and growth of cells or microorganisms.

In a specific embodiment, the protein:

(1) compared with the amino acid sequence as shown in SEQ ID NO: 1, comprises T6→V6 and/or N11→S11 mutations;

(2) compared with the amino acid sequence as shown in SEQ ID NO: 1, comprises one or more of T6→I6, N11→S11, R13→K13, I14→M14, E32→D32, P38→Q38 and Q59→K59 mutations;

(3) compared with the amino acid sequence as shown in SEQ ID NO: 1, comprises H4→G4 and/or T6→P6 mutations;

(4) compared with the amino acid sequence as shown in SEQ ID NO: 1, comprises one or more of H4→Q4, E7→Q7, N11→S11, E32→D32, P38→Q38, R53→K53 and 59Q→59K mutations;

(5) compared with the amino acid sequence as shown in SEQ ID NO: 1, comprises one or more of N11→S11, R13→K13, E32→D32, P38→Q38 and Q59→K59 mutations;

(6) compared with the amino acid sequence as shown in SEQ ID NO: 1, comprises one or more of H4→Q4, E7→Q7, N11→S11, A12→S12, R13→K13, E32→D32, P38→K38, R53→K53 and Q59→K59 mutations;

(7) compared with the amino acid sequence as shown in SEQ ID NO: 1, comprises one or more of H4→Q4, T6→I6, E7→H7, N11--S11, R13→K13, P16→S16, E32→D32, P38→K38, R53→K53 and Q59→K59 mutations;

(8) compared with the amino acid sequence as shown in SEQ ID NO: 1, comprises one or more of H4→Q4, E7→H7, N11→S11, R13→K13, E15→G15, P38→Q38, I49→V49, R53→K53 and Q59→K59 mutations;

(9) compared with the amino acid sequence as shown in SEQ ID NO: 1, comprises one or more of H4→Q4, E7→H7, N11→S11, R13→K13, E15→G15, E32→D32, P38→R38, I49→V49, R53→K53 and Q59→K59;

(10) compared with the amino acid sequence as shown in SEQ ID NO: 1, comprises one or more of H4→Q4, T6→I6, E7→H7, N11→S11, R13→K13, E32→D32, P38→K38, R53→K53 and Q59→K59 mutations;

(11) compared with the amino acid sequence as shown in SEQ ID NO: 1, comprises one or more of H4→K4, E7→Q7, E10→D10, N11→S11, E32→D32, P38→N38, R53→K53 and Q59→K59 mutations;

(12) compared with the amino acid sequence as shown in SEQ ID NO: 1, comprises one or more of H4→K4, N11→S11, R13→K13, E32→D32, P38→N38, R53→K53, R56→K56 and Q59→K59 mutations;

(13) compared with the amino acid sequence as shown in SEQ ID NO: 1, comprises one or more of H4→K4, T6→I6, E7→Q7, E10→D10, N11→S11, R13→K13, E32→D32, P38→N38, R53→K53 and Q59→K59 mutations;

(14) compared with the amino acid sequence as shown in SEQ ID NO: 1, comprises one or more of H4→K4, T6→N6, E7→Q7, E10→D10, N11→S11, R13→K13, E32→D32, P38→N38, R53→K53 and Q59→K59 mutations;

(15) compared with the amino acid sequence as shown in SEQ ID NO: 1, comprises one or more of H4→E4, T6→A6, E7→Q7, V9→M9, E10→D10, N11--S11, R13→K13, E32→D32, P38→N38, R53→K53 and Q59→K59 mutations;

(16) compared with the amino acid sequence as shown in SEQ ID NO: 1, comprises one or more of H4→Q4, E7→H7, N11→S11, R13→K13, E25→T25, E32→D32, P38→Q38, R53→K53 and Q59→K59 mutations;

(17) compared with the amino acid sequence as shown in SEQ ID NO: 1, comprises one or more of H4→K4, T6→P6, E7→Q7, E10→D10, N11→S11, R13→K13, E32→D32, P38→N38 and R53→K53, Q59→K59 mutations;

(18) compared with the amino acid sequence as shown in SEQ ID NO: 1, comprises one or more of H4→K4, E7→Q7, V9→M9, E10→D10, N11→S11, R13→K13, Y23→C23, E32→D32, P38→N38, R53→K53 and Q59→R59 mutations;

(19) compared with the amino acid sequence as shown in SEQ ID NO: 1, comprises one or more of H4→K4, N5→D5, T6→I6, E7→P7, P8→N8, N11→G11, A12→E12, R13→K13, P16→T16, K28→R28, E32→D32, P38→K38, K46→N46, R53→K53, R56→K56 and Q59→K59 mutations;

(20) compared with the amino acid sequence as shown in SEQ ID NO: 1, comprises one or more of H4→Q4, E7→Q7, N11→S11, R13→K13, E32→D32, P38→K38, R53→K53 and Q59→K59 mutations;

(21) compared with the amino acid sequence as shown in SEQ ID NO: 1, comprises one or more of H4→K4, N5→*del,* E7→Q7, E10→D10, N11→S11, R13→K13, E32→D32, P38→N38, R53→K53 and Q59→K59 mutations;

(22) compared with the amino acid sequence as shown in SEQ ID NO: 1, comprises one or more of H4→K4, N5→H5, E7→Q7, N11→S11, R13→K13, P16→A16, E32→D32, P38→N38, R53→K53 and Q59→K59 mutations;

(23) compared with the amino acid sequence as shown in SEQ ID NO: 1, comprises one or more of H4→K4, E7→Q7, E10→D10, N11→S11, R13→K13, D18→G18, E32→D32, P38→N38, H39→Y39, R53→K53 and Q59→K59 mutations;

(24) compared with the amino acid sequence as shown in SEQ ID NO: 1, comprises one or more of H4→Q4, N5→G5, T6→E6, E7→P7, P8→H8, N11→G11, R13→K13, P16→T16, E32→D32, P38→K38, K46→T46, R53→K53, R56→K56 and Q59→K59 mutations;

(25) compared with the amino acid sequence as shown in SEQ ID NO: 1, comprises one or more of H4→E4, T6→A6, E7→Q7, E10→D10, N11→S11, R13→K13, E32→D32, P38→N38, R53→K53 and Q59→K59 mutations;

(26) compared with the amino acid sequence as shown in SEQ ID NO: 1, comprises one or more of H4→K4, T6→N6, E7→P7, E10→D10, N11→S11, R13→K13, E32→D32, P38→N38, R53→K53 and Q59→K59 mutations;

(27) compared with the amino acid sequence as shown in SEQ ID NO: 1, comprises one or more of H4→K4, N5→S5, T6→P6, N11→S11, R13→K13, E32→D32, P38→K38, R53→K53 and Q59→K59 mutations;

(28) compared with the amino acid sequence as shown in SEQ ID NO: 1, comprises one or more of T6→V6, N11→S11, R13→K13, E32→D32, P38→Q38, S54→N54, Q59→K59 and L63→F63 mutations;

(29) compared with the amino acid sequence as shown in SEQ ID NO: 1, comprises one or more of H4→K4, N5→S5, T6→A6, E7→Q7, E10→D10, N11→S11, R13→K13, E32→D32, P38→S38, R53→K53 and Q59→K59 mutations;

(30) compared with the amino acid sequence as shown in SEQ ID NO: 1, comprises one or more of H4→K4, N5→A5, T6→A6, E7→Q7, E10→D10, N11→S11, R13→K13, E32→D32, P38→S38, R53→K53 and Q59→K59 mutations;

(31) compared with the amino acid sequence as shown in SEQ ID NO: 1, comprises T6→A6 and/or N11→S11 mutations;

(32) compared with the amino acid sequence as shown in SEQ ID NO: 1, comprises one or more of H4→Q4, N11→S11, R13→K13, E32→D32, P38→K38, R53→K53 and Q59→K59 mutations;

(33) compared with the amino acid sequence as shown in SEQ ID NO: 1, comprises one or more of H4→K4, N5→G5, T6→A6, E7→Q7, E10→D10, N11--S11, R13→K13, E32→D32, P38→N38, R53→K53 and Q59→K59 mutations;

(34) compared with the amino acid sequence as shown in SEQ ID NO: 1, comprises one or more of H4→K4, E7→H7, E10→D10, N11→S11, R13→K13, E32→D32, P38→N38, R53→K53 and Q59→K59 mutations;

(35) compared with the amino acid sequence as shown in SEQ ID NO: 1, comprises one or more of H4→K4, T6→A6, E7→Q7, E10→D10, N11→S11, R13→K13, E32→D32, P38→N38, R53→K53, R56→K56 and Q59→K59 mutations;

(36) compared with the amino acid sequence as shown in SEQ ID NO: 1, comprises one or more of H4→K4, T6→P6, E7→H7, E10→D10, N11→S11, R13→K13, E32→D32, P38→N38, R53→K53 and Q59→K59 mutations;

(37) compared with the amino acid sequence as shown in SEQ ID NO: 1, comprises one or more of H4→Q4, T6→I6, E7→*del,* N11→S11, R13→5K13, E32→D32, P38→K38, R53→K53 and Q59→K59 mutations;

(38) compared with the amino acid sequence as shown in SEQ ID NO: 1, comprises one or more of N11→S11, E15→D15, E32→D32, P38→Q38 and Q59→K59 mutations;

(39) compared with the amino acid sequence as shown in SEQ ID NO: 1, comprises one or more of H4→Q4, T6→I6, E7→H7, N11→S11, R13→K13, E15→D15, E32→D32, P38→K38, R53→K53 and Q59→K59 mutations;

(40) compared with the amino acid sequence as shown in SEQ ID NO: 1, comprises one or more of H4→Q4, E7→H7, N11→S11, R13→K13, E32→D32, P38→K38, R53→K53 and Q59→K59 mutations;

(41) compared with the amino acid sequence as shown in SEQ ID NO: 1, comprises one or more of H4→K4, N5→D5, E7→P7, P8→D8, N11→G11, A12→E12, R13→K13, E32→D32, P38→Q38, K46→N46, R53→K53, S54→N54, R56→K56 and Q59→K59 mutations;

(42) compared with the amino acid sequence as shown in SEQ ID NO: 1, comprises one or more of H4→Q4, N5→G5, T6→E6, E7→P7, P8→H8, N11→G11, R13→K13, P16→T16, E32→D32, P38→K38, K46→T46, R53→K53, R56→K56 and Q59→K59 mutations;

(43) compared with the amino acid sequence as shown in SEQ ID NO: 1, comprises one or more of H4→K4, T6→P6, E7→H7, V9→M9, E10→D10, N11→S11, R13→K13, E32→D32, P38→N38, R53→K53 and Q59→K59 mutations;

(44) compared with the amino acid sequence as shown in SEQ ID NO: 1, comprises one or more of H4→Q4, T6→16, E7→P7, N11→S11, R13→K13, E32→D32, P38→Q38, R53→K53, R56→K56 and Q59→K59 mutations;

(45) compared with the amino acid sequence as shown in SEQ ID NO: 1, comprises one or more of H4→K4, N5→T5, E7→Q7, E10→D10, N11→S11, R13→K13, E32→D32, P38→S38, R53→K53 and Q59→K59 mutations;

(46) compared with the amino acid sequence as shown in SEQ ID NO: 1, comprises one or more of H4→K4, E7→Q7, E10→D10, N11→S11, R13→K13, E32→D32, P38→N38, R53→K53 and Q59→K59 mutations;

(47) compared with the amino acid sequence as shown in SEQ ID NO: 1, comprises one or more of R53→K53 and/or Q59→K59 mutations;

(48) compared with the amino acid sequence as shown in SEQ ID NO: 1, has S added to the N-terminus, and comprises R53→K53 and/or Q59→K59 mutations;

(49) compared with the amino acid sequence as shown in SEQ ID NO: 1, has S added to the N-terminus and pentapeptide GLVPR to the C-terminus, and comprises R53→K53 and/or Q59→K59 mutations.

In a specific embodiment, the protein has no less than two, no less than three, no less than four or no less than five and no more than ten, no more than eleven, no more than twelve, no more than thirteen, no more than fourteen or no more than fifteen amino acid substitutions, deletions or additions compared with an amino acid sequence as shown in any one of SEQ ID NOs: 1-50.

Preferably, the protein comprises or is an amino acid sequence having at least 80%, at least 85%, at least 90%, at least 91%, at least 92%, at least 93%, at least 94%, at least 95%, at least 96%, at least 97%, at least 98%, at least 99% or 100% identity with an amino acid sequence as shown in any one of SEQ ID NOs: 1-50.

Preferably, the protein is an amino acid sequence as shown in any one of SEQ ID NOs: 2-50.

The second aspect of the present invention provides an isolated nucleic acid molecule, which comprises a nucleotide sequence encoding the protein.

The third aspect of the present invention provide an expression vector, which comprises the isolate nucleic acid molecule.

The fourth aspect of the present invention provides a culture medium supplement, which comprises at least one or a combination of the proteins.

The fifth aspect of the present invention provides a culture medium, which comprises at least one or a combination of the proteins or the supplement.

In a preferred embodiment, the working concentration of the protein is 0.02-1000 $\mu$g/mL, preferably 10-800 $\mu$g/mL, further preferably 20-600 $\mu$g/mL, more preferably 40-400 $\mu$g/mL or any concentration value between 0.02-1000 $\mu$g/mL; specifically, the working concentration of the protein is 0.02, 0.04, 0.1, 0.2, 0.4, 1, 2, 4, 10, 20, 40, 100, 200, 400, 600, 800, 1000 $\mu$g/mL.

Preferably, the medium further comprises a basal medium.

Preferably, the basal medium is selected from conventional culture media.

The sixth aspect of the present invention provides a method for culturing cells or microorganisms, which comprises the following steps:

providing cells or microorganisms for culture;
exposing the cells or the microorganisms to at least one or a combination of the proteins, the culture medium supplement or the culture medium; and
harvesting the culture.

Preferably, the cells are stem cells, cell lines or primary cells, and more preferably, the cell lines are genetically engineered cell lines.

The genetically engineered cell lines include, for example, Kit225, 293T and CHO; the primary cells include but are not limited to PMO and PBMC.

Preferably, the microorganisms are genetically engineered bacteria such as yeast and E. coli.

The seventh aspect of the present invention provides the use of the protein, the culture medium supplement or the culture medium in culturing cells or microorganisms.

Preferably, the cells are stem cells, cell lines or primary cells, and more preferably, the cell lines are genetically engineered cell lines.

The genetically engineered cell lines include, for example, Kit225, 293T and CHO; the primary cells include but are not limited to PMO and PBMC.

Preferably, the microorganisms are genetically engineered bacteria such as yeast and E. coli.

## BRIEF DESCRIPTION OF THE DRAWINGS

In order to explain the technical solutions of the present invention more clearly, the technical solutions of the present invention will be explained in detail with the drawings:

Fig. 1 shows the purification electrophoresis pattern of proteins with sample codes B01-B14;
Fig. 2 shows the purification electrophoresis pattern of proteins with sample codes B15-B28;
Fig. 3 shows the purification electrophoresis pattern of proteins with sample codes B29-B42;
Fig. 4 shows the purification electrophoresis pattern of proteins with sample codes B43-B47;
Fig. 5 shows the purification electrophoresis pattern of proteins with sample codes B81-B83;
Fig. 6 shows the effect of a protein with sample code B83 on the growth curve of KIT225 cells under nutrient-deficient conditions;
Fig. 7 shows the effect of a protein with sample code B83 on the survival rate of KIT225 cells under nutrient-deficient conditions;
Fig. 8 shows the effect of a protein with sample code B83 on the growth curve of 293T cells under nutrient-deficient conditions;
Fig. 9 shows the effect of a protein with sample code B83 on the survival rate of 293T cells under nutrient-deficient conditions;
Fig. 10 shows the effect of a protein with sample code B83 on the growth curve of CHO cells under nutrient-deficient conditions;
Fig. 11 shows the effect of a protein with sample code B83 on the survival rate of CHO cells under nutrient-deficient conditions;
Fig. 12 shows the effect of a protein with sample code B83 on the growth curve of PBMC cells under nutrient-deficient conditions;
Fig. 13 shows the effect of a protein with sample code B83 on the survival rate of PBMC cells under nutrient-deficient conditions;
Fig. 14 shows the effect of a protein with sample code B83 on the growth curve of PMO cells under nutrient-deficient conditions;

Fig. 15 shows the effect of a protein with sample code B83 on the survival rate of PMO cells under nutrient-deficient conditions.

## DETAILED DESCRIPTION OF THE INVENTION

The technical solutions of the present invention will be described below with specific test examples, but the scope of protection of the present invention is not limited thereto.

What is described in the test examples in the specification is only an enumeration of the implementations of the inventive concept, and the scope of protection of the present invention should not be regarded as being limited to the specific forms stated in the test examples, and the scope of protection of the present invention also covers equivalent technical means that can be thought of by those skilled in the art according to the concept of the present invention. Although the following embodiments of the present invention are described, the present invention is not limited to the specific embodiments and application fields, and the following specific embodiments are only illustrative, instructive and not restrictive. Those skilled in the art can make many forms under the inspiration of the present specification and without departing from the scope of the claims of the present invention, which are all within the scope of the protection of the present invention.

The following tests of the present invention are conclusive tests made by the research and development personnel on the basis of many creative tests and the technical solutions to be protected by the present invention. The quantitative tests in the following test examples are all arranged in triplicate, and the data are the mean or mean ± SD of the the three repeated tests.

Definitions

The amino acids forming the protein of the present invention can be natural or unnatural amino acids.

The expression "natural amino acids" refers to L-form amino acids found in natural proteins, namely: alanine (A), arginine (R), asparagine (N), aspartic acid (D), cysteine (C), glutamine (Q), glutamic acid (E), glycine (G), histidine (H), isoleucine (I), leucine (L), lysine (K), methionine (M), phenylalanine (F), proline (P), serine (S), threonine (T), tryptophan (W), tyrosine (Y) and valine (V).

The expression "unnatural amino acids" refers to the D-forms of natural amino acids, the homotypes of some natural amino acids (such as arginine, lysine, phenylalanine and serine), and the orthotypes of leucine and valine. It also includes synthetic amino acids, such as $\alpha$-aminobutyric acid (Abu), agmatine (Agm), alpha-arinoisobutyrique acid (Aib), N-formyl-Trp (F-trp), sarcosine, statine, ornithine and desaminotyrosine. Desaminotyrosine is incorporated into the N-terminus of these peptides, while agmatine and statine are incorporated into the C-terminus of these peptides.

In all discussions here, the standard single-letter code of amino acid is used, and the standard substitution notation is also used, where:

1) Amino acid residues are expressed in the form of single-letter symbols using the widely recognized IUPAC nomenclature. For DNA nucleic acid sequences, the widely recognized IUPAC nomenclature is also adopted.

2) Identification of mutant: "amino acids undergoing substitution at the original amino acid position" is used to represent the mutated amino acids in the mutant. For example, T6→V6 means that for the amino acid at position 6, threonine Thr is substituted with valine Val, and *T6→del* means that the amino acid at position 6 is deleted, and the numbering of the positions corresponds to the amino acid sequence numbering in SEQ ID NO: 1.

Cells

As used herein, the term "cell" refers to a single cell as well as a population (i.e. more than one cells). The population can be a pure population containing one type of cells, or can be one containing many types of cells. In the present invention, there is no limit to the number of cell types contained in a population. In addition, "cell culture" as used herein refers to the in vitro culture of any cell. This term encompasses continuous cell lines (i.e. with a permanent phenotype), primary cell culture, limited cell lines (i.e. non-transformed cells), and any other cell population maintained in vivo, including oocytes and embryos.

In some embodiments, the cells are of a non-genetically engineered cell line, while in other embodiments, the cells are of a genetically engineered cell line. In some embodiments, the cells contain genetically engineered molecules, which is not limited in the present invention, including any combination of cell types suitable for teratoma production or apoptosis detection, identification and/or quantification in samples, including mixed cell culture where all cell types are not genetically engineered cell lines; one or more cell types in the mixture are genetically engineered cell lines, while others are not; and all cell types in the mixture are genetically engineered cell lines.

As used herein, the term "primary cells" refers to cells obtained directly from tissues (e.g. blood) or organs of animals, including humans, without culture. Typically, though not necessarily, primary cells can be sub-cultured in vitro for 10 times or less before they die or their proliferation stops. On the contrary, "cultured cells" are cells that have been maintained and/or proliferated in vitro for 10 or more generations. Compared with primary cells of the same origin, cultured cells refer to cells that can be sub-cultured many times in vitro before they stop proliferation and/or die. Cultured cells include "cell lines" and "primary cultured cells".

The term "cell line" used herein refers to cells cultured in vitro, including primary cell lines, finite cell lines, continuous cell lines and transformed cell lines. This term does not require an infinite number of cultured cells. Cell lines can be produced spontaneously or by transformation.

As used herein, the terms "culture medium" and "cell culture medium" refer to media suitable for cell culture in vitro (i.e. cell culture). This term is not limited to any particular culture medium. For example, this definition includes not only maintenance media, but also by-products. Indeed, this term includes any culture medium suitable for cell growth.

The term "in vitro" as used herein refers to an artificial environment as well as the processes and reactions therein. The in vitro environment is exemplified by test tube and cell culture, but it is not limited thereto.

The term "in vivo" as used herein refers to a natural environment (i.e. animals or cells) and the processes and reactions therein.

The terms "proliferation" and "growth" as used herein can be used interchangeably to refer to the increase in the number of cells. On the contrary, maintenance refers to the continuous survival of cells or cell populations, but it is not necessarily survival with an increased number of cells.

The terms "stem cells", "cells without specific functions", "uncommitted cells" and "undifferentiated cells" as used herein mean that cells have a unique ability to renew themselves and produce specific types of cells, which constitute tissues and organs of the body. Embryonic stem cells have no tissue-specific structures and tissue-specific functions (i.e. cardiomyocytes, nerve cells, etc.). Stem cells can be derived from embryos (i.e. embryonic stem cells), fetal and adult tissues. The terms "specific", "committed" and "differentiated" refer to cells with tissue-specific structures and/or functions (i.e. cardiomyocytes, nerve cells, etc.). When the term "differentiation" is used to describe cells, it refers to a process by which cells without specific functions obtains specific functions (i.e. heart, liver or muscle cells).

**Examples**

The experimental methods for which specific conditions are not specified in the following examples are carried out according to the conventional methods and conditions, or according to the product instructions.

Plasmids pET30b and BL21 competent cells are purchased from Novagen;
Restriction endonucleases such as NdeI and HindIII, DNA marker, plasmid extraction kits and DNA gel recovery and purification kits are all purchased from Takara;
Superdex 200 molecular sieves and CM ion exchange chromatographic columns are purchased from NanoMicro;
All the chemical reagents are analytically pure domestic chemicals;
The operation steps of plasmid extraction make reference to the instructions of the plasmid mini extraction kit;
The operation steps of DNA gel recovery make reference to the instructions of the DNA gel recovery kit;
The operation steps of DNA fragment ligation make reference to the instructions of the T4 ligase;
LB medium: 5 g/L yeast powder, 10 g/L peptone, 10 g/L sodium chloride and the rest being water;
E. coli culture medium: 5 g/L yeast powder, 10 g/L peptone, 10 g/L sodium chloride and the rest being water;
Brewer's yeast culture medium: 20 g/L yeast powder, 40 g/L peptone, 40 g/L glucose and the rest being water;
Human T lymphocyte KIT225 cell line is purchased from ATCC;
Human renal epithelial cell line 293T is purchased from ATCC;
Hamster ovary cell CHO cell line is purchased from ATCC;
SD rats are purchased from Suzhou Elmet Biotechnology Co., Ltd.;
Culture medium 1640 is purchased from Gibco, with Art. No. 22400121;
FBS (fetal bovine serum) is purchased from Gibco, with Art. No. 10270-106;
HBSS culture medium is purchased from Beyotime, with Art. No. C0219.

**Example 1 Expression and purification of proteins**

The coding DNA sequence (not shown) of the target protein (as shown in SEQ ID NOs: 1-50) was obtained by gene synthesis. After double digestion with NdeI and HindIII, it was ligated to pET30b plasmid (Novagen) to give a recombinant plasmid expressing the target protein. After the recombinant plasmid was transformed into BL21 competent cells, positive clones were screened by plating, and 20 clones were selected and cultured in LB culture medium respectively. When $OD_{600}$ reached 0.6, 0.1 mM IPTG was added to induce expression, and it was verified whether the molecular weight meets the requirements. A series of recombinant strains were constructed by the above method, and the protein sequences expressed by the strains are shown in Table 1.

Table 1 Protein sequence

| SEQ ID NO: | Sample code | Amino acid sequence |
|---|---|---|
| 1 | B01 | TKVHVEPVESARIEPPDTGLYYDEYLXQVIEVLETDPHFREKLQKADIEEIRSGRLSQELDLVSH |
| 2 | B02 | TKVHVEPVESARIEPPDTGLYYDEYLXQVIEVLETDPHFREKLQKADIEEIRSGRLSQELDLVSH |
| 3 | B03 | TKVHVEPVESAKMEPPDTGLYYDEYLXQVIEVLETDPHFREKLQKADIEEIRSGRLSKELDLVSH |
| 4 | B04 | TKVGNPEPVESAKIDPPGLYYDEYLRQVIDVLETDNHFREKLQKADIEEIRSGKLSKELDLVSH |
| 5 | B05 | TKVQNTQPVESARIEPPDTGLYYDEYLRQVIDVLETDQHFREKLQKADIEEIRSGKLSKELDLVSH |
| 6 | B06 | TKVHNTEPVESAKIEPPDTGLYYDEYLKQVIDVLETDQHFREKLQKADIEEIRSGRLSKELDLVSH |
| 7 | B07 | TKVQNTQPVESSKIEPPDTGLYYDEYLRQVIDVLETDQHFREKLQKADIEEIRSGKLSKELDLVSH |
| 8 | B08 | TKVQNIHPVESAKIESPDTGLYYDEYLKQVIDVLETDKHFREKLQKADIEEIKSGRLSKEDLVSH |
| 9 | B09 | TKVQNTHPVESAKIGPPDTGLYYDEYLKQVIEVLETDQHFREKLQKADVEEIKSGRLSKEDLVSH |
| 10 | B10 | TKVQNTHPVESAKIGPPDTGLYYDEYLKQVIDVLETDQHFREKLQKADVEEIKSGRLSKEDLVSH |
| 11 | B11 | TKVQNIHPVESAKIGPPDTGLYYDEYLKQVIEVLETDQHFREKLQKADVEEIKSGRLSKEDLVSH |
| 12 | B12 | TKVKNTQPVDSARIEFPDTGLYYDEYLKQVIDVLETDNHFREKLQKADIEEIKSGRLSKELDLVSH |
| 13 | B13 | TKVKNTEPVDSARIEFPDTGLYYDEYLKQVIDVLETDNHFREKLQKADIEEIKSGRLSKELDLVSH |
| 14 | B14 | TKVKNIQPVDSAKIEPPDTGLYYDEYLKQVIDVLETDNHFREKLQKADIEEIKSGRESKELDLVSH |
| 15 | B15 | TKVKNTQPVDSAKIEFPDTGLYYDEYLKQVIDVLETDNHFREKLQKADIEEIKSGRLSKELDLVSH |
| 16 | B16 | TKVKNTQPMDSAKIEFPDTGLYYDEYLKQVIDVLETDNHFREKLQKADIEEIKSGRLSKELDLVSH |
| 17 | B17 | TKVQNTHPVESAKIEFPDTGLYYDEYLKQVIDVLETDQHFREKLQKADIEEIKSGRLSKELDLVSH |
| 18 | B18 | TKVQNTHPVESAKIEFPDTGLYYDEYLKQVIDVLETDQHFREKLQKADIEEIKSGRLSKELDLVSH |
| 19 | B19 | TKVQNTHPMDSAKIEFPDTGLYCDEYLKQVIDVLETDNHFREKLQKADIEEIKSGRLSKELDLVSH |
| 20 | B20 | TKVDIPNVEGEKIEFPDTGLYCDEYLKQVIDVLETDNHFREKLQKADIEEIKSGKLSKELDLVSH |
| 21 | B21 | TKVDIPNVEGEKIEPPDTGLYYDEYLKQVIDVIDVLETDKHLQKADIEEIKSGKLSKELDLVSH |
| 22 | B22 | TKVK-TQPVDSAK1EPPDGLYYDEYLKQV1DVLETDNHFREKLQKADIEEIKSGRLSKELDLVSH |
| 23 | B23 | TKVKHTQPVESAKIEAPDTGLYYDEYLKQVIDVLETDNHFREKLQKADIEEIKSGRLSKELDLVSH |
| 24 | B24 | TKVKNTQPVDSAKIFPPGTGLYYDEYLKQVIDVLETDNHFREKLQKADIEEIKSGRLSKELDLVSH |
| 25 | B25 | TKVQGEPHVEGAKIETPDTGLYYDEYLKQVIDVLETDKHFREKLQTADIEEIKSGKLSKELDLVSH |
| 26 | B26 | TKVENAQPVDSAKIEPPDTGLYYDEYLKQVIDVLETDNHFREKLQKADIEEIKSGRLSKELDLVSH |

(continued)

| SEQ ID NO: | Sample code | Amino acid sequence |
|---|---|---|
| 27 | B27 | TKVKNNPPVDSAKIEPPDTGLYYDEYLKQVIDVLETDNHFREKLQKADIEEIKSGRLSKELDLVSH |
| 28 | B28 | TKVKSPEPVESAKIEPPDTGLYYDEYLKQVIDVLETDKHFREKLQKADIEEIKSGRLSKELDLVSH |
| 29 | B29 | TKVHNVEPVESAKIEPPDTGLYYDEYLKQVIDVLETDQHFREKLQKADIEEIRNGRLSKELDFVSH |
| 30 | B30 | TKVKSAQPVDSAKIEPPDGLYYDEYLKQVIDVLETDSHFREKLQKADIEEIKSGRLSKFDLVSH |
| 31 | B31 | TKVKAAQPYDSAKIEPPDTGLYYDTYLKQVIDVLETDSIIFREKLQKADIEEIKSGRLSKLDLVSH |
| 32 | B32 | TKVHNAEPVESARIEPPDTGLYYDEYLKQVIEVLETDPHFREKLQKADIEEIRSGRLSQELDLVSH |
| 33 | B33 | TKVQNTEPVESAKIEPPDTGLYYDEYLKQVIDVIETDKHFREKLQKADIEEIKSGRLSKELDLVSH |
| 34 | B34 | TKVKGAQPVDSAKIEPPDTGLYYDEYLKQVIDVLETDNHFREKLQKADIEEIKSGRLSKELDLVSH |
| 35 | B35 | TKVKNTHPVDSAKIEPPDTGLYYDEYLKQVIDVLETDNHFREKLQKADIEEIKSGRLSKFLDLVSH |
| 36 | B36 | TKVKNAQPVDSAKIEPPDTGLYYDEYLKQVIDVLETDNHFREKLQKADIEEIKSGKLSKELDLVSH |
| 37 | B37 | TKVKNPHPVDSAKIEPPDTGLYYDEYLKQVIDETDXHFKLQKADIEEIKSGLSKELDVSH |
| 38 | B38 | TKVQNLPVESAKIEPPDTGLYYDEYLKQVIDVLETDKHFREKLQKADIEEIKSGRLSKELDLVSH |
| 39 | B39 | TKVHNTEPVESARIDPPDTGLYYDEYLKQVIDVLETDQHFREKLQKADIEEIRSGRLSKFLDLVSH |
| 40 | B40 | TKVQNTHPVESAKIDPPDTGLYYDFYLKQVIDVLETDKHFREKLQKADIEEIKSGRLSKELSLVSH |
| 41 | B41 | TKVQNTHPVESAKIEPPDTGLYYDEYLKQVIDVLETDKHFREKLQKADIEEIKSGRLSKELDLVSH |
| 42 | B42 | TKVKDTPDVEGEKTEPPDTGYYDEYLKQVIDVIETDQHFREKLQNADIEEIKNGKLSKELDLVSH |
| 43 | B43 | TKVQGEPHVGAKTPTPTGLYYDEYLKQVIDVLETDKHFREKLQTADIEEITKSGKLSKELDLVSH |
| 44 | B44 | TKYKNPIIPMDSAKIEPPDTGLYYDEYLKQVIDVLETDNHFREKLQKADIEEIKSGRLSKELDLVSH |
| 45 | B45 | TKVQNPPVESAKIEPPDTGLYYDEYLKQVIDVLETDQHFREKLQKADIEEIKSGKLSKELDLVSH |
| 46 | B46 | TKVKTTQPVDSAKIEPPDTGLYYDEYLKQVIDVLETDSFREKLQKADIEEIKSGRLSKELDLVSH |
| 47 | B47 | TKVKNTQPVDSAKIEPPDTGLYYDEYLKQVIDVLETDNHFREKLQKADIEEIKSGRLSKELDLVSH |
| 48 | B81 | TKVHNTEPVEVARIEYPPDTGLYYDEYLKQVIEVLETDPHFREKLQKADIEEIKSGRLSKELDLVSH |
| 49 | B82 | STKVHNTEPVENARIEPPDTGLYYDEYLKQVIEYLKQVIEVLETDPHFREKLQKADIEEIKSGRLSKELDLVSH |
| 50 | B83 | STKVHNTEPVENARIEPPDTGLYYDEYLKQVIEVLETDPHFREKLQKADIEEIKSGRLSKELDLVSHGLVPR |

Taking the preparation process of a sample with code B83 as an example, the strain was inoculated into a shake flask culture medium for large-scale expression, and the bacteria were collected after induction with IPTG. The bacteria were added into a buffer at a ratio of 1:10, and then ultrasonically crushed to give a bacterial lysate. The bacterial lysate was purified by the following steps:

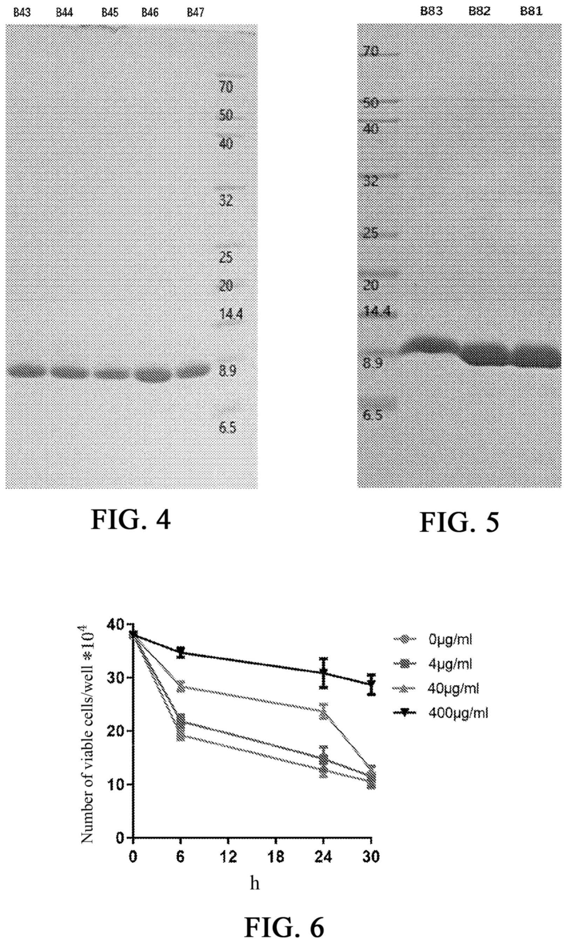

(1) the bacterial lysate was subjected to the first step of separation and purification using a CM ion exchange chromatographic column from NanoMicro; (2) the system was subjected to chromatography using a Superdex 200 molecular sieve from NanoMicro to give the target product (55 mg) with a purity of more than 95% and a molecular weight of 8,400 daltons (as shown in Fig. 5).

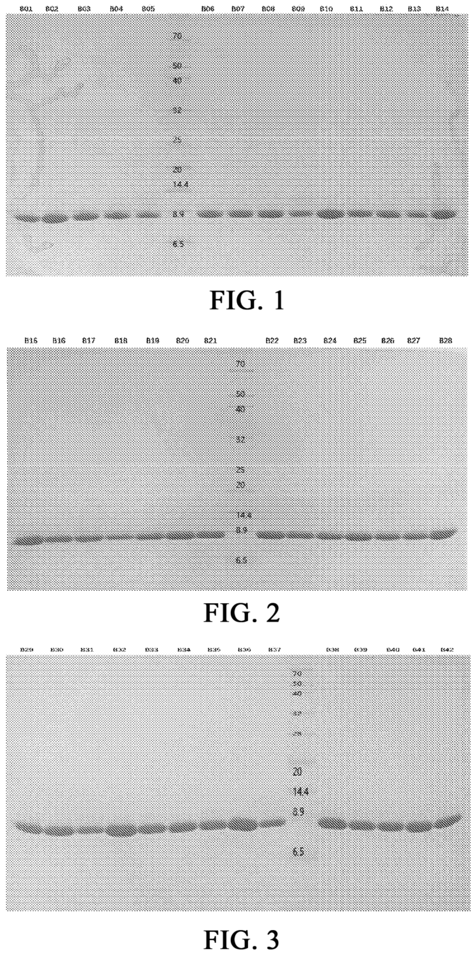

The preparation processes of samples with codes B1-B47, B81 and B82 are the same as that of B83, and the electrophoretic patterns of the resulting target products are shown in Fig. 1 to Fig. 5.

**Example 2 The protein of the present invention significantly improves the growth rate of E. coli.**

A culture medium for E. coli was prepared and sub-packaged into test tubes numbered 1-51. Parallel samples in triplicate were provided for each tube, and a control group was provided. All the test tubes were sterilized for later use.

More than 10 μM sterilized recombinant protein samples (codes B1-B47, B81, B82 and B83) were added respectively, and in the control group, an equal volume of sterile physiological saline was added.

All the groups were inoculated with E. coli bacterial solution at a ratio of 1:20. After inoculation, it was placed in a thermostatic shaker incubator and cultured at 37°C and 225 rpm. Samples were taken 2 hours later and $OD_{600}$ was recorded.

The results are shown in Table 2, which shows that the proteins of the present invention can all improve the 2h$OD_{600}$ of E. coli, especially the proteins indicated by codes B12, B14, B15, B16, B18, B23, B24, B26, B27, B30, B31, B34, B35, B37, B44, B46, B81, B82 and B83, among which B81, B82 and B83, especially B83, have particularly remarkable effects.

**Example 3 The protein of the present invention significantly increases the growth rate of brewer's yeast**

A culture medium for yeast growth was prepared, and sub-packaged into test tubes numbered 1-51. Parallel samples in triplicate were set for each tube, and a control group was provided. All test tubes were sterilized for later use.

10 μM sterilized recombinant protein samples (codes B1-B47, B81, B82 and B83) were added respectively, and in the control group, an equal volume of sterile physiological saline was added.

All groups were inoculated with the yeast culture at a ratio of 1:20. After inoculation, it was placed in a thermostatic shaker incubator and cultured at 37°C and 225 rpm. Samples were taken 2 hours later and $OD_{600}$ was recorded.

The results are shown in Table 2. It can be seen from Table 2 that these proteins can also increase the 2h$OD_{600}$ of brewer's yeast, especially the proteins indicated by codes B12, B14, B15, B16, B18, B23, B24, B26, B27, B30, B31, B34, B35, B37, B44, B46, B81, B82 and B83, among which B81, B82 and B83, especially B83, have particularly remarkable effects.

Table 2 $OD_{600}$ of E. coli and brewer's yeast

| Sample | 2h$OD_{600}$ of E. coli | | 2h$OD_{600}$ of brewer's yeast | |
|---|---|---|---|---|
| | Mean | SD | Mean | SD |
| Control | 0.92 | 0.25 | 0.66 | 0.26 |
| B01 | 1.01 | 0.20 | 0.72 | 0.31 |
| B02 | 0.95 | 0.31 | 0.67 | 0.28 |
| B03 | 1.12 | 0.46 | 0.73 | 0.22 |
| B04 | 1.19 | 0.34 | 0.90 | 0.25 |
| B05 | 1.41 | 0.30 | 1.14 | 0.29 |
| B06 | 1.14 | 0.43 | 0.78 | 0.30 |
| B07 | 1.39 | 0.41 | 1.09 | 0.34 |
| B08 | 1.35 | 0.24 | 1.08 | 0.32 |
| B09 | 1.42 | 0.23 | 1.02 | 0.27 |
| B10 | 1.44 | 0.47 | 1.23 | 0.36 |

(continued)

| Sample | 2hOD$_{600}$ of E. coli | | 2hOD$_{600}$ of brewer's yeast | |
|--------|------|------|------|------|
|        | Mean | SD   | Mean | SD   |
| B11 | 1.30 | 0.40 | 1.00 | 0.26 |
| B12 | 1.55 | 0.32 | 1.40 | 0.22 |
| B13 | 1.18 | 0.37 | 0.89 | 0.20 |
| B14 | 1.54 | 0.36 | 1.35 | 0.31 |
| B15 | 1.58 | 0.45 | 1.41 | 0.25 |
| B16 | 1.88 | 0.45 | 1.69 | 0.27 |
| B17 | 1.40 | 0.26 | 1.23 | 0.23 |
| B18 | 1.55 | 0.27 | 1.40 | 0.24 |
| B19 | 1.22 | 0.33 | 0.93 | 0.24 |
| B20 | 1.29 | 0.32 | 0.97 | 0.31 |
| B21 | 1.37 | 0.42 | 1.08 | 0.33 |
| B22 | 1.47 | 0.41 | 1.24 | 0.20 |
| B23 | 1.84 | 0.42 | 1.60 | 0.36 |
| B24 | 1.82 | 0.37 | 1.60 | 0.31 |
| B25 | 1.25 | 0.37 | 0.93 | 0.36 |
| B26 | 1.86 | 0.42 | 1.62 | 0.24 |
| B27 | 1.58 | 0.20 | 1.45 | 0.33 |
| B28 | 1.42 | 0.46 | 1.21 | 0.20 |
| B29 | 1.10 | 0.41 | 0.73 | 0.26 |
| B30 | 1.82 | 0.48 | 1.60 | 0.24 |
| B31 | 1.80 | 0.45 | 1.56 | 0.24 |
| B32 | 0.96 | 0.23 | 0.68 | 0.33 |
| B33 | 1.39 | 0.32 | 1.11 | 0.23 |
| B34 | 1.65 | 0.23 | 1.46 | 0.31 |
| B35 | 1.71 | 0.27 | 1.49 | 0.24 |
| B36 | 1.20 | 0.47 | 0.91 | 0.30 |
| B37 | 1.66 | 0.28 | 1.47 | 0.24 |
| B38 | 1.29 | 0.49 | 0.96 | 0.20 |
| B39 | 1.12 | 0.40 | 0.75 | 0.31 |
| B40 | 1.34 | 1.34 | 1.06 | 1.06 |
| B41 | 1.34 | 0.36 | 1.07 | 0.26 |
| B42 | 1.28 | 0.33 | 0.95 | 0.21 |
| B43 | 1.23 | 0.28 | 0.93 | 0.20 |
| B44 | 1.78 | 0.20 | 1.49 | 0.29 |
| B45 | 1.15 | 0.36 | 0.85 | 0.23 |
| B46 | 1.60 | 0.42 | 1.44 | 0.39 |
| B47 | 1.49 | 0.20 | 1.29 | 0.34 |
| B81 | 1.92 | 0.39 | 1.67 | 0.39 |

(continued)

| Sample | 2hOD$_{600}$ of E. coli | | 2hOD$_{600}$ of brewer's yeast | |
|---|---|---|---|---|
| | Mean | SD | Mean | SD |
| B82 | 1.93 | 0.34 | 1.69 | 0.21 |
| B83 | 1.95 | 0.27 | 1.77 | 0.35 |

**Example 4 The protein of the present invention significantly improves the survival rate of human T lymphocyte KIT225 cell line under nutrient-deficient conditions**

1. Cell line resuscitation: 1) Culture medium 1640:FBS=90:10 was prepared, and 3 mL of the culture medium was taken and placed in a 15 mL centrifuge tube; 2) the cells were taken out of liquid nitrogen and melted in a water bath at 37°C for 1 min; 3) a little amount of the culture medium was added to a cell cryogenic tube to transfer the cells to the 15 mL centrifuge tube; 4) the cells were centrifuged at 1000×g for 3 min, the supernatant was removed, 4 mL of the culture medium was slowly added and the system was mixed thoroughly by blowing; 5) the cells were evenly transferred into a culture dish by a pipette, and then transferred into an incubator.
2. Cell line passage: 1) The old culture medium in the culture dish was collected to a 15 mL centrifuge tube; 2) the system was transferred to a 15 mL centrifuge tube, centrifuged at 1000×g for 3 min, the supernatant was removed, 4 mL of the culture medium was added, the system was mixed thoroughly by blowing, and sub-cultured at 1:4.
3. Cell line plating: 1) The old culture medium in the culture dish was collected to a 15 mL centrifuge tube; 2) the culture medium was centrifuged at 1000×g for 3 min; 3) the supernatant was removed, HBSS culture medium was added, and the system was gently mixed by blowing; 4) the cells were plated to a 24-well plate according to the following experimental grouping requirements.

They were divided into four groups: protein B83 was added to final concentrations of 0 μg/mL, 4 μg/mL, 40 μg/mL and 400 μg/mL respectively, with 3 wells for each group, 380,000 cells/well, with a volume of 1 mL/ well. The cells were cultured for 6 h, 24 h and 30 h, sampled, and stained with trypan blue to detect the cell survival rate.

$$\text{Cell survival rate} = (\text{total number of cells} - \text{number of blue cells})/\text{total number of cells} * 100\%$$

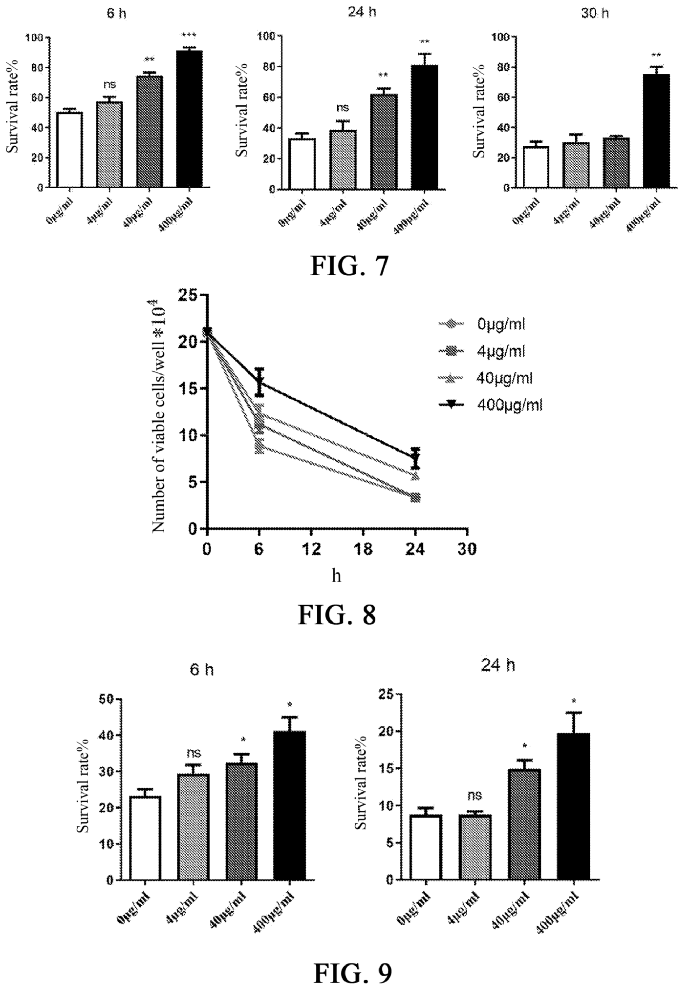

The results are as shown in Fig. 6 and Fig. 7, the number of viable KIT225 cells in nutrient-deficient HBSS decreased obviously with the passage of time. However, after B83 was added, the number of viable cells increased obviously at the same time point, indicating that the survival rate was obviously improved. Especially, the working concentration of 400 μg/mL significantly improved the cell survival rate.

**Example 5 The protein of the present invention significantly improves the survival rate of human renal epithelial cell line 293T under nutrient-deficient conditions**

1. Cell line resuscitation: 1) Culture medium 1640:FBS=90:10 was prepared, and 3 mL of the culture medium was taken and placed in a 15 mL centrifuge tube; 2) the cells were taken out of liquid nitrogen and melted in a water bath at 37°C for 1 min; 3) a little amount of the culture medium was added to a cell cryogenic tube to transfer the cells to the 15 mL centrifuge tube; 4) the cells were centrifuged at 1000×g for 3 min, the supernatant was removed, 4 mL of the culture medium was slowly added and the system was mixed thoroughly by blowing; 5) the cells were evenly transferred into a culture dish by a pipette, and then transferred into an incubator.
2. Cell line passage: 1) The old culture medium in the culture dish was collected to a 15 mL centrifuge tube; 2) the system was transferred to a 15 mL centrifuge tube, centrifuged at 1000×g for 3 min, the supernatant was removed, 4 mL of the culture medium was added, the system was mixed thoroughly by blowing, and sub-cultured at 1:4.
3. Cell line plating: 1) The old culture medium in the culture dish was collected to a 15 mL centrifuge tube; 2) the culture medium was centrifuged at 1000×g for 3 min; 3) the supernatant was removed, HBSS culture medium was added, and the system was gently mixed by blowing; 4) the cells were plated to a 24-well plate according to the following experimental grouping requirements.

They were divided into four groups: protein B83 was added to final concentrations of 0 μg/mL, 4 μg/mL, 40 μg/mL and 400 μg/mL respectively, with 3 wells for each group, 380,000 cells/well, with a volume of 1 mL/ well. The cells were cultured for 6 h and 24 h, sampled, and stained with trypan blue to detect the cell survival rate.

$$\text{Cell survival rate} = (\text{total number of cells - number of blue cells})/\text{total number of cells} * 100\%$$

The results are shown in Fig. 8 and 9, the number of viable 293T cells in nutrient-deficient HBSS decreased obviously with the passage of time. However, after B83 was added, the number of viable cells increased obviously at the same time point, indicating that the survival rate was obviously improved. Especially, the working concentration of 400 μg/mL significantly improved the cell survival rate.

**Example 6 The protein of the present invention significantly improves the survival rate of hamster ovary cell CHO cell line under nutrient-deficient conditions**

1. Cell line resuscitation: 1) Culture medium 1640:FBS=90:10 was prepared, and 3 mL of the culture medium was taken and placed in a 15 mL centrifuge tube; 2) the cells were taken out of liquid nitrogen and melted in a water bath at 37°C for 1 min; 3) a little amount of the culture medium was added to a cell cryogenic tube to transfer the cells to the 15 mL centrifuge tube; 4) the cells were centrifuged at 1000×g for 3 min, the supernatant was removed, 4 mL of the culture medium was slowly added and the system was mixed thoroughly by blowing; 5) the cells were evenly transferred into a culture dish by a pipette, and then transferred into an incubator.

2. Cell line passage: 1) The old culture medium in the culture dish was collected to a 15 mL centrifuge tube; 2) the system was transferred to a 15 mL centrifuge tube, centrifuged at 1000×g for 3 min, the supernatant was removed, 4 mL of the culture medium was added, the system was mixed thoroughly by blowing, and sub-cultured at 1:4.

3. Cell line plating: 1) The old culture medium in the culture dish was collected to a 15 mL centrifuge tube; 2) the culture medium was centrifuged at 1000×g for 3 min; 3) the supernatant was removed, HBSS culture medium was added, and the system was gently mixed by blowing; 4) the cells were plated to a 24-well plate according to the following experimental grouping requirements.

They were divided into four groups: protein B83 was added to final concentrations of 0 μg/mL, 4 μg/mL, 40 μg/mL and 400 μg/mL respectively, with 3 wells for each group, 380,000 cells/well, with a volume of 1 mL/ well. The cells were cultured for 6 h and 24 h, sampled, and stained with trypan blue to detect the cell survival rate.

$$\text{Cell survival rate} = (\text{total number of cells - number of blue cells})/\text{total number of cells} * 100\%$$

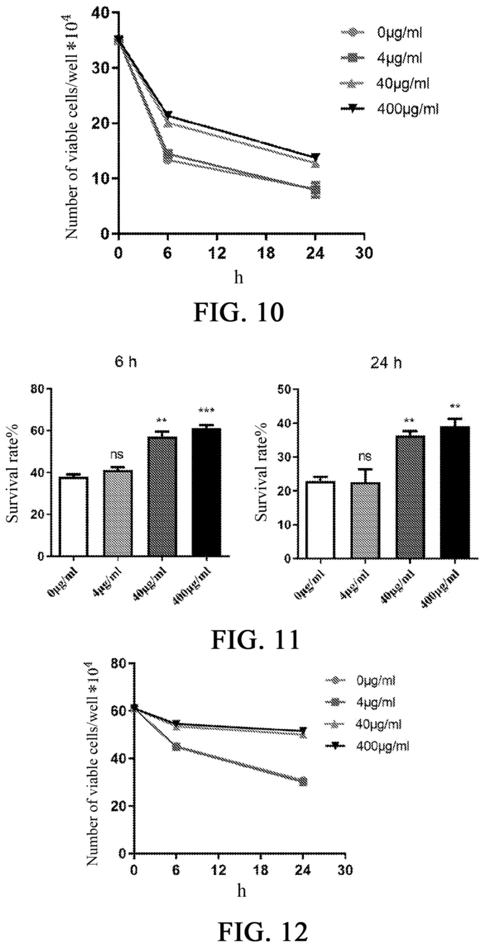

The results are as shown in Fig. 10 and Fig. 11, the number of viable CHO cells in nutrient-deficient HBSS decreased obviously with the passage of time. However, after B83 was added, the number of viable cells increased obviously at the same time point, indicating that the survival rate was obviously improved. Especially, the working concentration of 40-400 μg/mL significantly improved the cell survival rate.

**Example 7 The protein of the present invention significantly improves the survival rate of primary peritoneal macrophages (PMOs) of rat under nutrient-deficient conditions**

1. Extraction and culture of rat peritoneal macrophages: In the first three days of the experiment, 3% sodium thioglycollate or starch broth (5 mL per rat) was injected into the abdominal cavity, and then the following steps were implemented: 1) The rat was killed by cervical dislocation, soaked in 75% alcohol for 1-2 min, transferred to a super clean bench, placed on a dissection table, its limbs were fixed with needles, and the skin was teared and pulled to the two sides with forceps in both hands to expose the peritoneum, but without damaging the peritoneal wall. 2) After the peritoneal wall was scrubbed with 70% alcohol, 20 mL of a serum-free culture medium was injected into the abdominal cavity with a syringe, and at the same time the peritoneal wall was kneaded with the fingers from both sides to make the liquid flow thoroughly in the abdominal cavity. 3) The abdominal wall was gently lifted with a needle, so that the animal's body leaned slightly to one side to make the liquid in the abdominal cavity to accumulate beneath the needle and the liquid was sucked into the needle tube. 4) The needle was carefully pulled out, the liquid was injected into a centrifuge tube and centrifuged at 4°C for 10 min at 1200 r/min, the supernatant was discarded, the process was repeated twice, and a complete culture medium was added for re-suspension. 5) The cells were counted by the trypan blue exclusion assay, the cells were diluted to the target concentration, and then inoculated. 6) If used as feeder cells, the cells was adjusted to a concentration of $2\times10^5$/mL and inoculated into a 96-well plate, with 100-200 $\mu$L per well; if used in other experiments, the cells can be adjusted to $2\times10^6$/mL and added to a 24-well flat-bottom culture plate, with 1 mL/well; after incubation in a 5% $CO_2$ incubator for 12 h, the medium was replaced, the cells were washed with RPMI1640 medium for 1-2 times, the non-adherent cells (red blood cells) were discarded, and the adherent cells were single-layer PMO cells.

2. Cell plating: 1) The cells in the culture dish was collected to a 15 mL centrifuge tube; 2) the cells were centrifuged at $1000\times$g for 3 min; 3) the supernatant was removed, HBSS culture medium was added, and the system was gently mixed by blowing; 4) the cells were plated to a 24-well plate according to the following experimental grouping requirements.

They were divided into four groups: protein B83 was added to final concentrations of 0 $\mu$g/mL, 4 $\mu$g/mL, 40 $\mu$g/mL and 400 $\mu$g/mL respectively, with 3 wells for each group, 380,000 cells/well, with a volume of 1 mL/ well. The cells were cultured for 6 h and 24 h, sampled, and stained with trypan blue to detect the cell survival rate.

$$\text{Cell survival rate} = (\text{total number of cells - number of blue cells})/\text{total number of cells} * 100\%$$

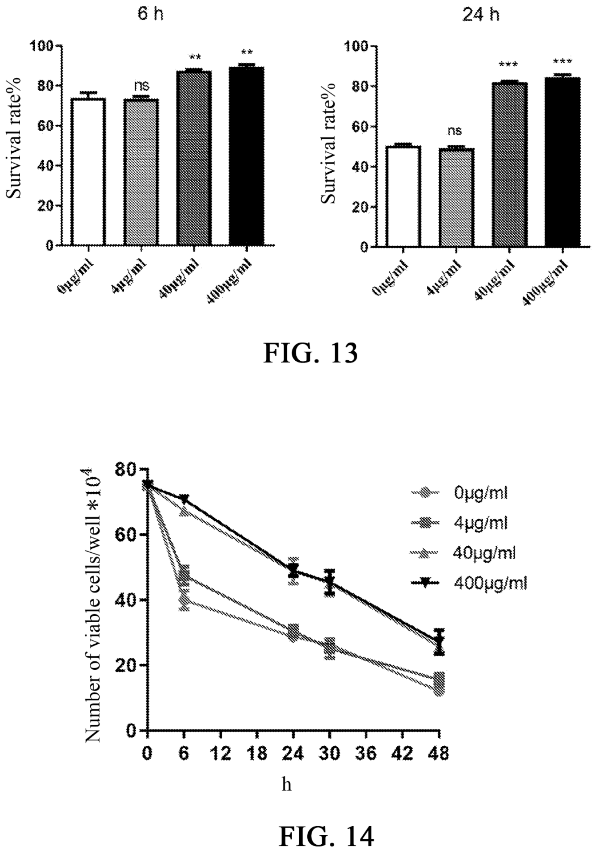

The results are as shown in Fig. 12 and Fig. 13, the number of viable PMO cells in nutrient-deficient HBSS decreased obviously with the passage of time. However, after B83 was added, the number of viable cells increased obviously at the same time point, indicating that the survival rate was obviously improved. Especially, the working concentration of 40- 400 $\mu$g/mL significantly improved the cell survival rate

**Example 8 The protein of the present invention significantly improves the survival rate of primary peripheral blood mononuclear cells (PBMCs) of rat under nutrient-deficient conditions**

1. Isolation of rat peripheral blood mononuclear cells (PBMCs): (1) 10 mL of whole blood was transferred into a 50 mL centrifuge tube, 10 ml of PBS solution was added for dilution, and the system was gently mixed; (2) Two 15 mL centrifuge tubes were taken, 5 mL of lymphocyte separation medium was added first, then the diluted blood was gently added to the upper layer of lymphocyte separation medium in the two centrifuge tubes, 10 mL of diluted blood per centrifuge tube, the operation must be gentle to avoid mixing the two solutions; (3) The system was centrifuged at 2,000 rpm for 30 min; it should be noted that the deceleration setting must be set as no break, or with only 10-20% braking; (4) The cell layer where PBMC is located was white, and at this time, this layer of cells can be transferred into another clean 15 mL centrifuge tube with a syringe; (5) PBS was added to 10-15 mL, the mixture was centrifuged at 1,500 rpm for 10 min, the supernatant was discarded, and then the culture medium was added to carry out the same procedure of washing, and the resultant cells were PBMCs.
2. Cell plating: The PBMC cells were re-suspended with 5-10 mL of 1640 culture medium, followed by counting, culture or plating.

They were divided into four groups: protein B83 was added to final concentrations of 0 $\mu$g/mL, 4 $\mu$g/mL, 40 $\mu$g/mL and 400 $\mu$g/mL respectively, with 3 wells for each group, 380,000 cells/well, with a volume of 1 mL/ well. The cells were cultured for 6 h, 24 h, 36 h and 48 h, sampled, and stained with trypan blue to detect the cell survival rate.

$$\text{Cell survival rate} = (\text{total number of cells - number of blue cells})/\text{total number of cells} * 100\%$$

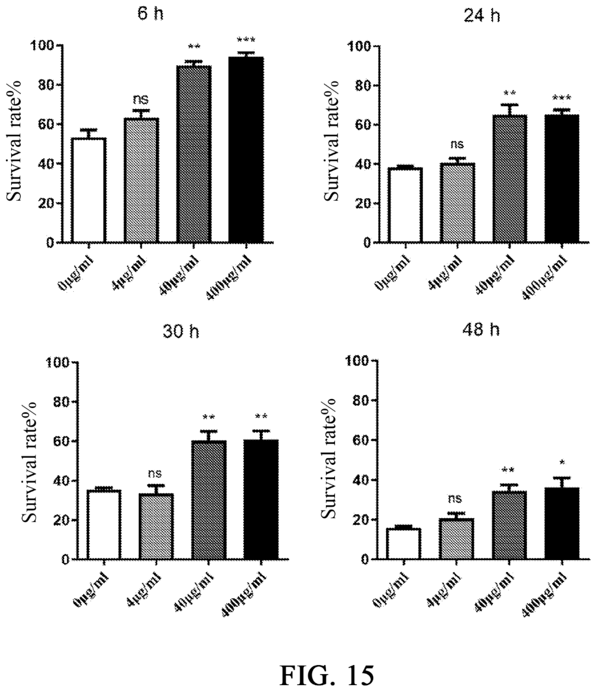

The results are as shown in Fig. 14 and Fig. 15, the number of viable PBMC cells in nutrient-deficient HBSS decreased obviously with the passage of time. However, after B83 was added, the number of viable cells increased obviously at the same time point, indicating that the survival rate was obviously improved. Especially, the working concentration of 40- 400 $\mu$g/mL significantly improved the cell survival rate.

For those skilled in the art, many improvements and supplements can be made without departing from the method of the present invention, and these improvements and supplements should also be regarded as the scope of protection of the present invention.

**Claims**

1. A protein, being a variant of an amino acid sequence as shown in SEQ ID NO: 1, wherein the variant comprises at least one of the following mutations:

   (a) S is added to the N-terminus;
   (b) position 4 is H, G, Q, K or E;
   (c) position 5 is D, H, G, S, A, T or is absent;
   (d) position 6 is V, I, P, N, A or E;
   (e) position 7 is Q, H, P or is absent;
   (f) position 8 is N, H or D;
   (g) position 9 is M;
   (h) position 10 is D;
   (i) position 11 is S or G;
   (j) position 12 is S or E;
   (k) position 13 is K;
   (l) position 14 is M;
   (m) position 15 is G or D;
   (n) position 16 is S, T or A;
   (o) position 18 is G;
   (p) position 23 is C;
   (q) position 25 is T;
   (r) position 28 is R;
   (s) position 32 is D;
   (t) position 38 is Q, K, R, N or S;
   (u) position 39 is Y;
   (v) position 46 is N or T;
   (w) position 49 is V;
   (x) position 53 is K;
   (y) position 54 is N;
   (z) position 56 is K;
   (aa) position 59 is K or R;
   (bb) position 63 is F; and/or
   (cc) the C-terminus is pentapeptide GLVPR or is absent.

2. The protein according to claim 1, wherein the protein:

   (1) compared with the amino acid sequence as shown in SEQ ID NO: 1, comprises T6→V6 and/or N11→S11 mutations;
   (2) compared with the amino acid sequence as shown in SEQ ID NO: 1, comprises one or more of T6→I6, N11→S11, R13→K13, I14→M14, E32→D32, P38→Q38 and Q59→K59 mutations;
   (3) compared with the amino acid sequence as shown in SEQ ID NO: 1, comprises H4→G4 and/or T6→P6 mutations;
   (4) compared with the amino acid sequence as shown in SEQ ID NO: 1, comprises one or more of H4→Q4, E7→Q7, N11→S11, E32→D32, P38→Q38, R53→K53 and 59Q→59K mutations;
   (5) compared with the amino acid sequence as shown in SEQ ID NO: 1, comprises one or more of N11→S11, R13→K13, E32→D32, P38→Q38 and Q59→K59 mutations;
   (6) compared with the amino acid sequence as shown in SEQ ID NO: 1, comprises one or more of H4→Q4, E7→Q7, N11→S11, A12→S12, R13→K13, E32→D32, P38→K38, R53→K53 and Q59→K59 mutations;
   (7) compared with the amino acid sequence as shown in SEQ ID NO: 1, comprises one or more of H4→Q4, T6→I6, E7→H7, N11→S11, R13→K13, P16→S16, E32→D32, P38→K38, R53→K53 and Q59→K59 mutations;
   (8) compared with the amino acid sequence as shown in SEQ ID NO: 1, comprises one or more of H4→Q4, E7→H7, N11→S11, R13→K13, E15→G15, P38→Q38, I49→V49, R53→K53 and Q59→K59 mutations;
   (9) compared with the amino acid sequence as shown in SEQ ID NO: 1, comprises one or more of H4→Q4, E7→H7, N11→S11, R13→5K13, E15→G15, E32→D32, P38→R38, I49→V49, R53→K53 and Q59→K59;
   (10) compared with the amino acid sequence as shown in SEQ ID NO: 1, comprises one or more of H4→Q4, T6→I6, E7→H7, N11→S11, R13→K13, E32→D32, P38→K38, R53→K53 and Q59→K59 mutations;
   (11) compared with the amino acid sequence as shown in SEQ ID NO: 1, comprises one or more of H4→K4, E7→Q7, E10→D10, N11→S11, E32→D32, P38→N38, R53→K53 and Q59→K59 mutations;

(12) compared with the amino acid sequence as shown in SEQ ID NO: 1, comprises one or more of H4→K4, N11→S11, R13→K13, E32→D32, P38→N38, R53→K53, R56→K56 and Q59→K59 mutations;

(13) compared with the amino acid sequence as shown in SEQ ID NO: 1, comprises one or more of H4→K4, T6→I6, E7→Q7, E10→D10, N11--S11, R13→K13, E32→D32, P38→N38, R53→K53 and Q59→K59 mutations;

(14) compared with the amino acid sequence as shown in SEQ ID NO: 1, comprises one or more of H4→K4, T6→N6, E7→Q7, E10→D10, N11→S11, R13→K13, E32→D32, P38→N38, R53→K53 and Q59→K59 mutations;

(15) compared with the amino acid sequence as shown in SEQ ID NO: 1, comprises one or more of H4→E4, T6→A6, E7→Q7, V9→M9, E10→D10, N11→S11, R13→K13, E32→D32, P38→N38, R53→K53 and Q59→K59 mutations;

(16) compared with the amino acid sequence as shown in SEQ ID NO: 1, comprises one or more of H4→Q4, E7→H7, N11→S11, R13→K13, E25→T25, E32→D32, P38→Q38, R53→K53 and Q59→K59 mutations;

(17) compared with the amino acid sequence as shown in SEQ ID NO: 1, comprises one or more of H4→K4, T6→P6, E7→Q7, E10→D10, N11→S11, R13→K13, E32→D32, P38→N38 and R53→K53, Q59→K59 mutations;

(18) compared with the amino acid sequence as shown in SEQ ID NO: 1, comprises one or more of H4→K4, E7→Q7, V9→M9, E10→D10, N11→S11, R13→K13, Y23→C23, E32→D32, P38→N38, R53→K53 and Q59→R59 mutations;

(19) compared with the amino acid sequence as shown in SEQ ID NO: 1, comprises one or more of H4→K4, N5→D5, T6→I6, E7→P7, P8→N8, N11→G11, A12→E12, R13→K13, P16→T16, K28→R28, E32→D32, P38→K38, K46→N46, R53→K53, R56→K56 and Q59→K59 mutations;

(20) compared with the amino acid sequence as shown in SEQ ID NO: 1, comprises one or more of H4→Q4, E7→Q7, N11→S11, R13→K13, E32→D32, P38→K38, R53→K53 and Q59→K59 mutations;

(21) compared with the amino acid sequence as shown in SEQ ID NO: 1, comprises one or more of H4→K4, *N5→del,* E7→Q7, E10→D10, N11→S11, R13→K13, E32→D32, P38→N38, R53→K53 and Q59→K59 mutations;

(22) compared with the amino acid sequence as shown in SEQ ID NO: 1, comprises one or more of H4→K4, N5→H5, E7→Q7, N11→S11, R13→K13, P16→A16, E32→D32, P38→N38, R53→K53 and Q59→K59 mutations;

(23) compared with the amino acid sequence as shown in SEQ ID NO: 1, comprises one or more of H4→K4, E7→Q7, E10→D10, N11→S11, R13→K13, D18→G18, E32→D32, P38→N38, H39→Y39, R53→K53 and Q59→K59 mutations;

(24) compared with the amino acid sequence as shown in SEQ ID NO: 1, comprises one or more of H4→Q4, N5→G5, T6→E6, E7→P7, P8→H8, N11→G11, R13→K13, P16→T16, E32→D32, P38→K38, K46→T46, R53→K53, R56→K56 and Q59→K59 mutations;

(25) compared with the amino acid sequence as shown in SEQ ID NO: 1, comprises one or more of H4→E4, T6→A6, E7→Q7, E10→D10, N11→S11, R13→K13, E32→D32, P38→N38, R53→K53 and Q59→K59 mutations;

(26) compared with the amino acid sequence as shown in SEQ ID NO: 1, comprises one or more of H4→K4, T6→N6, E7→P7, E10→D10, N11→S11, R13→5K13, E32→D32, P38→N38, R53→K53 and Q59→K59 mutations;

(27) compared with the amino acid sequence as shown in SEQ ID NO: 1, comprises one or more of H4→K4, N5→S5, T6→P6, N11→S11, R13→K13, E32→D32, P38→K38, R53→K53 and Q59→K59 mutations;

(28) compared with the amino acid sequence as shown in SEQ ID NO: 1, comprises one or more of T6→V6, N11→S11, R13→K13, E32→D32, P38→Q38, S54→N54, Q59→K59 and L63→F63 mutations;

(29) compared with the amino acid sequence as shown in SEQ ID NO: 1, comprises one or more of H4→K4, N5→S5, T6→A6, E7→Q7, E10→D10, N11→S11, R13→K13, E32→D32, P38→S38, R53→K53 and Q59→K59 mutations;

(30) compared with the amino acid sequence as shown in SEQ ID NO: 1, comprises one or more of H4→K4, N5→A5, T6→A6, E7→Q7, E10→D10, N11--S11, R13→K13, E32→D32, P38→S38, R53→K53 and Q59→K59 mutations;

(31) compared with the amino acid sequence as shown in SEQ ID NO: 1, comprises T6→A6 and/or N11→S11 mutations;

(32) compared with the amino acid sequence as shown in SEQ ID NO: 1, comprises one or more of H4→Q4, N11→S11, R13→K13, E32→D32, P38→K38, R53→K53 and Q59→K59 mutations;

(33) compared with the amino acid sequence as shown in SEQ ID NO: 1, comprises one or more of H4→K4, N5→G5, T6→A6, E7→Q7, E10→D10, N11→S11, R13→K13, E32→D32, P38→N38, R53→K53 and Q59→K59 mutations;

(34) compared with the amino acid sequence as shown in SEQ ID NO: 1, comprises one or more of H4→K4, E7→H7, E10→D10, N11→S11, R13→K13, E32→D32, P38→N38, R53→K53 and Q59→K59 mutations;

(35) compared with the amino acid sequence as shown in SEQ ID NO: 1, comprises one or more of H4→K4, T6→A6, E7→Q7, E10→D10, N11→S11, R13→K13, E32→D32, P38→N38, R53→K53, R56→K56 and Q59→K59 mutations;

(36) compared with the amino acid sequence as shown in SEQ ID NO: 1, comprises one or more of H4→K4, T6→P6, E7→H7, E10→D10, N11→S11, R13→K13, E32→D32, P38→N38, R53→K53 and Q59→K59 mutations;

(37) compared with the amino acid sequence as shown in SEQ ID NO: 1, comprises one or more of H4→Q4, T6→I6, *E7→del,* N11→S11, R13→K13, E32→D32, P38→K38, R53→K53 and Q59→K59 mutations;

(38) compared with the amino acid sequence as shown in SEQ ID NO: 1, comprises one or more of N11→S11, E15→D15, E32→D32, P38→Q38 and Q59→K59 mutations;

(39) compared with the amino acid sequence as shown in SEQ ID NO: 1, comprises one or more of H4→Q4, T6→I6, E7→H7, N11--S11, R13→K13, E15→D15, E32→D32, P38→K38, R53→K53 and Q59→K59 mutations;

(40) compared with the amino acid sequence as shown in SEQ ID NO: 1, comprises one or more of H4→Q4, E7→H7, N11→S11, R13→K13, E32→D32, P38→K38, R53→K53 and Q59→K59 mutations;

(41) compared with the amino acid sequence as shown in SEQ ID NO: 1, comprises one or more of H4→K4, N5→D5, E7→P7, P8→D8, N11→G11, A12→E12, R13→K13, E32→D32, P38→Q38, K46→N46, R53→K53, S54→N54, R56→K56 and Q59→K59 mutations;

(42) compared with the amino acid sequence as shown in SEQ ID NO: 1, comprises one or more of H4→Q4, N5→G5, T6→E6, E7→P7, P8→H8, N11→G11, R13→K13, P16→T16, E32→D32, P38→K38, K46→T46, R53→K53, R56→K56 and Q59→K59 mutations;

(43) compared with the amino acid sequence as shown in SEQ ID NO: 1, comprises one or more of H4→K4, T6→P6, E7→H7, V9→M9, E10→D10, N11→S11, R13→K13, E32→D32, P38→N38, R53→K53 and Q59→K59 mutations;

(44) compared with the amino acid sequence as shown in SEQ ID NO: 1, comprises one or more of H4→Q4, T6→I6, E7→P7, N11→S11, R13→K13, E32→D32, P38→Q38, R53→K53, R56→K56 and Q59→K59 mutations;

(45) compared with the amino acid sequence as shown in SEQ ID NO: 1, comprises one or more of H4→K4, N5→T5, E7→Q7, E10→D10, N11→S11, R13→K13, E32→D32, P38→S38, R53→K53 and Q59→K59 mutations;

(46) compared with the amino acid sequence as shown in SEQ ID NO: 1, comprises one or more of H4→Q4, E7→Q7, E10→D10, N11→S11, R13→K13, E32→D32, P38→N38, R53→K53 and Q59→K59 mutations;

(47) compared with the amino acid sequence as shown in SEQ ID NO: 1, comprises one or more of R53→K53 and/or Q59→K59 mutations;

(48) compared with the amino acid sequence as shown in SEQ ID NO: 1, has S added to the N-terminus, and comprises R53→K53 and/or Q59→K59 mutations;

(49) compared with the amino acid sequence as shown in SEQ ID NO: 1, has S added to the N-terminus and pentapeptide GLVPR to the C-terminus, and comprises R53→K53 and/or Q59→K59 mutations.

3. The protein according to claim 1 or 2, wherein the protein is a sequence with no less than two, no less than three, no less than four or no less than five and no more than ten, no more than eleven, no more than twelve, no more than thirteen, no more than fourteen or no more than fifteen amino acid substitutions, deletions or additions as compared with an amino acid sequence as shown in any one of SEQ ID NOs: 1-50.

   preferably, the protein comprises or is an amino acid sequence having at least 80%, at least 85%, at least 90%, at least 91%, at least 92%, at least 93%, at least 94%, at least 95%, at least 96%, at least 97%, at least 98%, at least 99% or 100% identity with an amino acid sequence as shown in any one of SEQ ID NOs: 1-50;
   preferably, the protein is an amino acid sequence as shown in any one of SEQ ID NOs: 2-50.

4. An isolated nucleic acid molecule, comprising a nucleotide sequence encoding the protein according to any one of claims 1 to 3.

5. An expression vector, comprising the isolated nucleic acid molecule according to claim 4.

6. A culture medium supplement, comprising at least one or a combination of the proteins according to any one of claims 1 to 3.

7. A culture medium, comprising at least one or a combination of the proteins according to any one of claims 1 to 3, or the supplement according to claim 6.

8. The culture medium supplement according to claim 6 or the culture medium according to claim 7, wherein the culture medium is a liquid culture medium, and/or

   in the culture medium, the working concentration of the protein is 0.02-1000 μg/mL, preferably 10-800 μg/mL, further preferably 20-600 μg/mL, and more preferably 40-400 μg/mL;

preferably, the culture medium further comprises a basal medium.

9. A method for culturing cells or microorganisms, comprising the following steps:

providing cells or microorganisms for culture;
exposing the cells or the microorganisms to at least one or a combination of the proteins according to any one of claims 1 to 3, the culture medium supplement according to claim 6 or the culture medium according to claim 7, and harvesting the culture;
preferably, the cells are stem cells, cell lines or primary cells, more preferably, the cell lines are genetically engineered cell lines;
preferably, the microorganisms are engineering bacteria, more preferably yeast or E. coli.

10. Use of the protein according to any one of claims 1 to 3, the culture medium supplement according to claim 6 or the culture medium according to claim 7 in culturing cells or microorganisms.

FIG. 1

FIG. 2

FIG. 3

FIG. 4

FIG. 5

FIG. 6

FIG. 7

FIG. 8

FIG. 9

FIG. 10

FIG. 11

FIG. 12

**FIG. 13**

**FIG. 14**

FIG. 15

## INTERNATIONAL SEARCH REPORT

| International application No. |
|---|
| **PCT/CN2024/086601** |

**A. CLASSIFICATION OF SUBJECT MATTER**

C07K 14/00(2006.01)i;  C12N 5/00(2006.01)i;  C12N1/20(2006.01)i

According to International Patent Classification (IPC) or to both national classification and IPC

**B. FIELDS SEARCHED**

Minimum documentation searched (classification system followed by classification symbols)

IPC: C07K; C12N

Documentation searched other than minimum documentation to the extent that such documents are included in the fields searched

Electronic data base consulted during the international search (name of data base and, where practicable, search terms used)

CNTXT, USTXT, WOTXT, VEN, CNABS, PubMed, ISI Web of Knowledge, CNKI, NCBI, CJFD, baidu, STN, 中国专利生物序列检索, China Patent Biological Sequence Search: SEQ ID NOs: 1 and 50, nucleobindin, 变体, 培养基, 促进生长, 细胞, 微生物, NUCB肽, GLVPR, mutant, 核结合素, medium, promote growth, cell, microorganism, 张菁

**C. DOCUMENTS CONSIDERED TO BE RELEVANT**

| Category* | Citation of document, with indication, where appropriate, of the relevant passages | Relevant to claim No. |
|---|---|---|
| A | CN 101914150 A (LIU, Jianning) 15 December 2010 (2010-12-15)<br>sequence listing: sequences 18 and 23 | 1-10 (in part) |
| A | WO 2010009330 A1 (THE ROCKEFELLER UNIVERSITY et al.) 21 January 2010 (2010-01-21)<br>abstract | 1-10 (in part) |
| A | US 2011224143 A1 (THE ROCKEFELLER UNIVERSITY et al.) 15 September 2011 (2011-09-15)<br>entire document | 1-10 (in part) |
| A | CN 109153971 A (FUJIFILM CORP.) 04 January 2019 (2019-01-04)<br>entire document | 1-10 (in part) |
| A | US 2014005105 A1 (LANDING BIOTECH, INC.) 02 January 2014 (2014-01-02)<br>entire document | 1-10 (in part) |
| A | MURAL, R.J. et al. "Nucleobindin 2, Isoform CRA_b[Mus musculus]"<br>*Genbank: EDL17109.1*, 26 July 2016 (2016-07-26),<br>entire document | 1-10 (in part) |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **04 July 2024** | **10 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

| **INTERNATIONAL SEARCH REPORT** | International application No. |
| | **PCT/CN2024/086601** |

| Box No. I          Nucleotide and/or amino acid sequence(s) (Continuation of item 1.c of the first sheet) |
| --- |

1.  With regard to any nucleotide and/or amino acid sequence disclosed in the international application, the international search was carried out on the basis of a sequence listing:

    a.   ☑  forming part of the international application as filed.

    b.   ☐  furnished subsequent to the international filing date for the purposes of international search (Rule 13*ter*.1(a)),

          ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
| --- |
| **PCT/CN2024/086601** |

**Box No. III       Observations where unity of invention is lacking (Continuation of item 3 of first sheet)**

This International Searching Authority found multiple inventions in this international application, as follows:

Group 1: Claims 1-10 (in part) relate to a protein, which contains mutations of T6→V6 and/or N11→S11 compared to the amino acid sequence shown in SEQ ID NO:1, a nucleic acid molecule encoding the protein, an expression vector comprising the nucleic acid molecule, a culture medium supplement comprising the protein, a culture medium, a cell or microorganism culture method, and the use of the protein.

Group 2: Claims 1-10 (in part) relate to a protein, which contains one or more mutations of T6→I6, N11→S11, R13→K13, I14→M14, E32→D32, P38→Q38 and Q59→K59 compared to the amino acid sequence shown in SEQ ID NO:1, a nucleic acid molecule encoding the protein, an expression vector comprising the nucleic acid molecule, a culture medium supplement comprising the protein, a culture medium, a cell or microorganism culture method, and the use of the protein.

Groups 3-49: Claims 1-10 (in part) relate to a protein, which, compared to the amino acid sequence shown in SEQ ID NO:1, contains (3) mutations of H4→G4 and/or T6→P6, (4) one or more mutations of H4→Q4, E7→Q7, N11→S11, E32→D32, P38→Q38, R53→K53 and 59Q→59K, (5) one or more mutations of N11→S11, R13→K13, E32→D32, P38→Q38 and Q59→K59, (6) one or more mutations of H4→Q4, E7→Q7, N11→S11, A12→S12, R13→K13, E32→D32, P38→K38, R53→K53 and Q59→K59, (7) one or more mutations of H4→Q4, T6→I6, E7→H7, N11→S11, R13→K13, P16→S16, E32→D32, P38→K38, R53→K53 and Q59→K59, (8) one or more mutations of H4→Q4, E7→H7, N11→S11, R13→K13, E15→G15, P38→Q38, I49→V49, R53→K53 and Q59→K59, (9) one or more mutations of H4→Q4, E7→H7, N11→S11, R13→K13, E15→G15, E32→D32, P38→R38, I49→V49, R53→K53 and Q59→K59, (10) one or more mutations of H4→Q4, T6→I6, E7→H7, N11→S11, R13→K13, E32→D32, P38→K38, R53→K53 and Q59→K59, (11) one or more mutations of H4→K4, E7→Q7, E10→D10, N11→S11, E32→D32, P38→N38, R53→K53 and Q59→K59, (12) one or more mutations of H4→K4, N11→S11, R13→K13, E32→D32, P38→N38, R53→K53, R56→K56 and Q59→K59, (13) one or more mutations of H4→K4, T6→I6, E7→Q7, E10→D10, N11→S11, R13→K13, E32→D32, P38→N38, R53→K53 and Q59→K59, (14) one or more mutations of H4→K4, T6→N6, E7→Q7, E10→D10, N11→S11, R13→K13, E32→D32, P38→N38, R53→K53 and Q59→K59, (15) one or more mutations of H4→E4, T6→A6, E7→Q7, V9→M9, E10→D10, N11→S11, R13→K13, E32→D32, P38→N38, R53→K53 and Q59→K59, (16) one or more mutations of H4→Q4, E7→H7, N11→S11, R13→K13, E25→T25, E32→D32, P38→Q38, R53→K53 and Q59→K59, (17) one or more mutations of H4→K4, T6→P6, E7→Q7, E10→D10, N11→S11, R13→K13, E32→D32, P38→N38, R53→K53 and Q59→K59, (18) one or more mutations of H4→K4, E7→Q7, V9→M9, E10→D10, N11→S11, R13→K13, Y23→C23, E32→D32, P38→N38, R53→K53 and Q59→R59, (19) one or more mutations of H4→K4, N5→D5, T6→I6, E7→P7, P8→N8, N11→G11, A12→E12, R13→K13, P16→T16, K28→R28, E32→D32, P38→K38, K46→N46, R53→K53, R56→K56 and Q59→K59, (20) one or more mutations of H4→Q4, E7→Q7, N11→S11, R13→K13, E32→D32, P38→K38, R53→K53 and Q59→K59, (21) one or more mutations of H4→K4, N5→del, E7→Q7, E10→D10, N11→S11, R13→K13, E32→D32, P38→N38, R53→K53 and Q59→K59, (22) one or more mutations of H4→K4, N5→H5, E7→Q7, N11→S11, R13→K13, P16→A16, E32→D32, P38→N38, R53→K53 and Q59→K59, (23) one or more mutations of H4→K4, E7→Q7, E10→D10, N11→S11, R13→K13, D18→G18, E32→D32, P38→N38, H39→Y39, R53→K53 and Q59→K59, (24) one or more mutations of H4→Q4, N5→G5, T6→E6, E7→P7, P8→H8, N11→G11, R13→K13, P16→T16, E32→D32, P38→K38, K46→T46, R53→K53, R56→K56 and Q59→K59, (25) one or more mutations of H4→E4, T6→A6, E7→Q7, E10→D10, N11→S11, R13→K13, E32→D32, P38→N38, R53→K53 and Q59→K59, (26) one or more mutations of H4→K4, T6→N6, E7→P7, E10→D10, N11→S11, R13→K13, E32→D32, P38→N38, R53→K53 and Q59→K59, (27) one or more mutations of H4→K4, N5→S5, T6→P6, N11→S11, R13→K13, E32→D32, P38→K38, R53→K53 and Q59→K59, (28) one or more mutations of T6→V6, N11→S11, R13→K13, E32→D32, P38→Q38, S54→N54, Q59→K59 and L63→F63, (29) one or more mutations of H4→K4, N5→S5, T6→A6, E7→Q7, E10→D10, N11→S11, R13→K13, E32→D32, P38→S38, R53→K53 and Q59→K59, (30) one or more mutations of H4→K4, N5→A5, T6→A6, E7→Q7, E10→D10, N11→S11, R13→K13, E32→D32, P38→S38, R53→K53 and Q59→K59, (31) mutations of T6→A6 and/or N11→S11, (32) one or more mutations of H4→Q4, N11→S11, R13→K13, E32→D32, P38→K38, R53→K53 and Q59→K59, (33) one or more mutations of H4→K4, N5→G5, T6→A6, E7→Q7, E10→D10, N11→S11, R13→K13, E32→D32, P38→N38, R53→K53 and Q59→K59, (34) one or more mutations of H4→K4, E7→H7, E10→D10, N11→S11, R13→K13, E32→D32, P38→N38, R53→K53 and Q59→K59, (35) one or more mutations of H4→K4, T6→A6, E7→Q7, E10→D10, N11→S11, R13→K13, E32→D32, P38→N38, R53→K53, R56→K56 and Q59→K59, (36) one or more mutations of H4→K4, T6→P6, E7→H7, E10→D10, N11→S11, R13→K13, E32→D32, P38→N38, R53→K53 and Q59→K59, (37) one or more mutations of H4→Q4, T6→I6, E7→del, N11→S11, R13→K13, E32→D32, P38→K38, R53→K53 and Q59→K59, (38) one or more mutations of N11→S11, E15→D15, E32→D32, P38→Q38 and Q59→K59, (39)

Form PCT/ISA/210 (continuation of first sheet) (July 2022)

## INTERNATIONAL SEARCH REPORT

International application No.

**PCT/CN2024/086601**

| Box No. III | Observations where unity of invention is lacking (Continuation of item 3 of first sheet) |
| --- | --- |

one or more mutations of H4→Q4, T6→I6, E7→H7, N11→S11, R13→K13, E15→D15, E32→D32, P38→K38, R53→K53 and Q59→K59, (40) one or more mutations of H4→Q4, E7→H7, N11→S11, R13→K13, E32→D32, P38→K38, R53→K53 and Q59→K59, (41) one or more mutations of H4→K4, N5→D5, E7→P7, P8→D8, N11→G11, A12→E12, R13→K13, E32→D32, P38→Q38, K46→N46, R53→K53, S54→N54, R56→K56 and Q59→K59, (42) one or more mutations of H4→Q4, N5→G5, T6→E6, E7→P7, P8→H8, N11→G11, R13→K13, P16→T16, E32→D32, P38→K38, K46→T46, R53→K53, R56→K56 and Q59→K59, (43) one or more mutations of H4→K4, T6→P6, E7→H7, V9→M9, E10→D10, N11→S11, R13→K13, E32→D32, P38→N38, R53→K53 and Q59→K59, (44) one or more mutations of H4→Q4, T6→I6, E7→P7, N11→S11, R13→K13, E32→D32, P38→Q38, R53→K53, R56→K56 and Q59→K59, (45) one or more mutations of H4→K4, N5→T5, E7→Q7, E10→D10, N11→S11, R13→K13, E32→D32, P38→S38, R53→K53 and Q59→K59, (46) one or more mutations of H4→K4, E7→Q7, E10→D10, N11→S11, R13→K13, E32→D32, P38→N38, R53→K53 and Q59→K59, (47) one or more mutations of R53→K53 and/or Q59→K59, and (48) an N-terminus added with S, and mutations of R53→K53 and/or Q59→K59, or (49) an N-terminus added with S, a C-terminus added with pentapeptide GLVPR, and mutations of R53→K53 and/or Q59→K59; and a nucleic acid molecule encoding the protein, an expression vector comprising the nucleic acid molecule, a culture medium supplement comprising the protein, a culture medium, a cell or microorganism culture method, and the use of the protein.

The same or corresponding technical feature among the 49 groups of inventions is "a variant of the amino acid sequence as shown in SEQ ID NO: 1". However, this technical feature is disclosed in the prior art. Therefore, the 49 groups of inventions do not have a same or corresponding special technical feature that defines a contribution which the inventions make over the prior art, do not fall within a single general inventive concept, and therefore do not comply with the requirement of unity of invention and do not comply with PCT Rule 13.1.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-10 (in part), which relate to an amino acid sequence variant having an N-terminus added with S and a C-terminus added with pentapeptide GLVPR, and comprising mutations of R53→K53 and/or Q59→K59 compared to the amino acid sequence shown in SEQ ID NO:1**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)

**INTERNATIONAL SEARCH REPORT**
Information on patent family members

International application No.

**PCT/CN2024/086601**

| Patent document cited in search report | | | Publication date (day/month/year) | Patent family member(s) | | | Publication date (day/month/year) |
|---|---|---|---|---|---|---|---|
| CN | 101914150 | A | 15 December 2010 | EP | 2229952 | A1 | 22 September 2010 |
| | | | | EP | 2229952 | B1 | 13 August 2014 |
| | | | | US | 2010235935 | A1 | 16 September 2010 |
| | | | | US | 8541367 | B2 | 24 September 2013 |
| WO | 2010009330 | A1 | 21 January 2010 | US | 2011224143 | A1 | 15 September 2011 |
| | | | | US | 8703707 | B2 | 22 April 2014 |
| US | 2011224143 | A1 | 15 September 2011 | WO | 2010009330 | A1 | 21 January 2010 |
| | | | | US | 8703707 | B2 | 22 April 2014 |
| CN | 109153971 | A | 04 January 2019 | US | 2019144525 | A1 | 16 May 2019 |
| | | | | WO | 2017200039 | A1 | 23 November 2017 |
| | | | | JPWO | 2017200039 | A1 | 22 March 2019 |
| | | | | EP | 3460050 | A4 | 27 March 2019 |
| | | | | EP | 3460050 | A1 | 03 November 2021 |
| US | 2014005105 | A1 | 02 January 2014 | US | 9018159 | B2 | 28 April 2015 |

Form PCT/ISA/210 (patent family annex) (July 2022)